# EUROPEAN PATENT SPECIFICATION

(11) **EP 3 034 090 B1**
(45) Date of publication and mention of the grant of the patent: **14.05.2025**
(21) Application number: 14836643.8
(22) Date of filing: 31.07.2014
(51) Int. Cl.: A61K 38/26, A61K 38/22, A61K 9/08, A61P 3/10, A61P 3/04, A61K 9/00, A61K 47/10, A61K 47/12, A61K 47/18, A61K 47/26, A61K 47/02, A61K 47/40, A61K 47/36

(54) **STABLE INSULIN SECRETAGOGUE PEPTIDE HYDRO-INJECTION PHARMACEUTICAL COMPOSITION**
STABILE PHARMAZEUTISCHE ZUSAMMENSETZUNG ZUR INSULIN-SEKRETIONSFÖRDERER-PEPTID-HYDRO-INJEKTION
COMPOSITION PHARMACEUTIQUE STABLE POUR L'HYDRO-INJECTION DE PEPTIDE SÉCRÉTAGOGUE D'INSULINE

(30) Priority: 13.08.2013 CN 201310351740
(43) Date of publication of application: 22.06.2016
(73) Proprietor: Shanghai Benemae Pharmaceutical Corporation, Shanghai 201321 (CN)
(72) Inventor: XIONG, Chunlin, Shanghai 201321 (CN); HE, Yunxia, Shanghai 201321 (CN); ZUO, Yajun, Shanghai 201321 (CN); YU, Gang, Shanghai 201321 (CN)
(74) Representative: Gulde & Partner
(86) International application number: PCT/CN2014/083370
(87) International publication number: WO 2015/021861

(56) References cited:
- EP-A1- 1 600 162
- WO-A1-2011/109787
- CN-A- 101 342 365
- CN-A- 101 466 394
- CN-A- 102 363 633
- CN-A- 102 405 055
- CN-A- 102 772 787
- CN-A- 103 405 753
- US-A1- 2002 061 838
- US-A1- 2005 143 303
- US-A1- 2006 263 849
- NOVONORDISK: "Product Information Victoza_pi5a.docx", 15 May 2013 (2013-05-15), XP055386064, Retrieved from the Internet <URL:www.novonordisk.com.au/content/dam/australia/affiliate/www-novonordisk-au/Health Care Professionals/Documents/Victoza_pi5a_MarketingVersion.pdf> [retrieved on 20170628]

## Description

### Technical Field

The present invention relates to an insulinotropic peptide multi-dose aqueous parenteral pharmaceutical composition and use thereof.

### Background Art

Glucagon-like peptide 1 (also designated GLP-1) and Exendin are both insulinotropic peptides, and have 53% identity in amino acid sequences thereof. Pharmacology has proven that both GLP-1 and Exendin-4 act on GLP-1 receptors of insulin-secreting β TC1 cells. This type of hormones can promote insulin secretion, and exert a glucose concentration-dependent hypoglycemic effect.

Similar to insulin, GLP-1 and Exendin are effective only when injected before meals. However, because a protein or polypeptide molecule is unstable, it cannot be developed to an oral pharmaceutical composition and must be used by injection. Even if a drug under development is in an injectable form, it tends to be a lyophilized injection powder that is inconvenient to use.

Instability of proteins and polypeptides includes two general aspects, i.e., physical and chemical aspects. Physical instability includes, for example, denaturation, surface adsorption, aggregation, precipitation, gelatination, and the like, and chemical instability includes, for example, hydrolysis, deamination, oxidization, racemization, isomerization, β-elimination, disulfide bond exchange, and the like. Such a series of unstabilizing factors will all change with the alteration in structures of the proteins or polypeptides, and therefore many protein or polypeptide drugs are produced by employing a freezing-drying method, such that the use of the formulation can meet the requirements for shelf life.

However, the production of lyophilized injection powders by employing the freezing-drying method suffers from many disadvantages: for example, high production cost, inconvenient for patients (the injection powder is in single doses, and prior to use each time, the patient needs to dissolve the injection powder with water, draw the mixture from a Penicillin bottle, and then injected the mixture), i.e., poor conformability, and therefore the market competitiveness is poor. Therefore, the development of multi-dose parenteral solutions not only provides convenience to patients, but also reduces the production cost, and thus has rather important significance for the improvement of market competitiveness.

Insulinotropic peptides, in particular GLP-1, have properties of these polypeptides, particularly physical instability, such as formation of gel, and therefore, in order to develop successfully multi-dose aqueous parenteral pharmaceutical compositions, these physical and chemical instability described above must be solved, to allow the compositions to achieve the pharmaceutically available period of validity.

In the development of multi-dose aqueous parenteral pharmaceutical compositions, adding a preservative into the pharmaceutical composition must be taken into consideration, so as to ensure that there is no microbial contamination during the storage duration and the usage period. However, most of preservatives are harmful to proteins or polypeptides, and interact with the proteins to make them unstable, leading to aggregation. For example, phenol preservatives, such as metacresol and phenol, cause human growth hormones to aggregate (Kirsch et al, 1993), phenol allows β-folds in insulin-type drugs to increase, and benzyl alcohol allows recombinant human interferon-γ to aggregate. Therefore, in the screening of pharmaceutical compositions, the relationship between the antimicrobial effect of the preservative and stability of the protein or polypeptide should be balanced. The trickiest difficulty in the development of parenteral solutions is to allow the formula to be able to be stored for 2 years or more at 4°C after addition of an preservative. Many raw material drugs or stock solutions of proteins or polypeptides have no problem in storage for 2 years or more at 4°C, but have difficulty in meeting the requirements for shelf life after the addition of the preservative, just because the addition of the preservative will severely influence stability of the drug.

Dissolution enhancers selected for most proteins or polypeptides are surfactants and PEG. Surfactants are mostly Tween, Span, Poloxamer, Pluronic, Brij and the like. In addition to these substances, surfactants selected in the present invention further include propylene glycol and dextran. Propylene glycol and dextran have very good effects when used as GLP-1 dissolution enhancers.

Patent WO00/37098 filed by Brader, Mark, L. also mentions GLP-1 instability: physical stability is poor between pH 6.8 and 7.5, the formulation will become turbid at a pH value less than 8.0 after the addition of an preservative, and chemical stability is reduced when the pH value is greater than 8.8, therefore the suitable pH range is narrow, pH 8.2 to 8.8. A range claimed therein is 0.3 mg/mL to 0.65 mg/mL, and a particularly stable concentration is 0.5 mg/mL. They carried out the work employing synthetic GLP-1, which also indicates that GLP-1 is unstable indeed.

In US 2005/143303 A1 a pharmaceutical composition of the prior art is disclosed which comprises GLP-1 and is intended for intranasal administration.

In US2002/0061838 A1 an injectable pharmaceutical composition is disclosed which comprises mannitol and acetate buffer.

Whereas the present invention provides a aqueous parenteral pharmaceutical composition that allows GLP-1 to have greater stability, and the aqueous parenteral pharmaceutical composition has a GLP-1 concentration far higher than the drug concentration achievable by the prior art.

### Summary of the Invention

The present invention is directed to subject matter as defined in the claims. In particular, the present invention is directed to subject matter of sole independent claim 1 referring to
a stable insulinotropic peptide multi-dose injectable aqueous parenteral pharmaceutical composition that can be stored for a long term, the aqueous parenteral pharmaceutical composition comprising an insulinotropic peptide, mannitol, propylene glycol, phenol and a pharmaceutically acceptable buffer salt solution of sodium acetate-acetic acid (NaAc-HAc);
wherein, the aqueous parenteral pharmaceutical composition has a pH value of 3 to 5; the insulinotropic peptide has a concentration of 0.1 mg/mL to 20 mg/mL, and preferably 0.5 mg/mL to 5 mg/mL; and
wherein the insulinotropic peptide is GLP-1.

In one aspect, the present invention provides an insulinotropic peptide multi-dose aqueous parenteral pharmaceutical composition that can be stored for a long term and have greater stability. The formulated aqueous parenteral pharmaceutical composition can meet the requirement for storage duration. The aqueous parenteral pharmaceutical composition comprises an insulinotropic peptide, a pharmaceutically acceptable isotonic agent, a pharmaceutically acceptable dissolution enhancer, a pharmaceutically acceptable preservative and a pharmaceutically acceptable buffer salt. Wherein, the aqueous parenteral pharmaceutical composition has a pH value of 3 to 5.

In the aqueous parenteral pharmaceutical composition of the present invention, the insulinotropic peptide is GLP-1.

In the present application, the term "analogue" serves to denote such a peptide wherein one or more amino acid residues of the parent peptide have been substituted with other amino acid residues and/or wherein one or more amino acid residues of the parent peptide have been deleted and/or wherein one or more amino acid residues have been added into the parent peptide. This addition may be occurred at N-terninus or C-terminal or both, of the parent peptide. In general, an "analogue" is such a peptide wherein six or less amino acids of the parent peptide have been substituted and/or added and/or deleted, more preferably is such a peptide wherein three or less amino acids of the parent peptide have been substituted and/or added and/or deleted, and most preferably is such a peptide wherein one amino acid of the parent peptide have been substituted and/or added and/or deleted.

In the present application, the term "derivative" serves to denote such a peptide wherein one or more amino acid residues of the parent peptide have a substituent introduced therein, and typical variants of the substituent include amide, sugars, alkyl, acyl, ester, PEGylation and the like.

The insulinotropic peptide is GLP-1.

Preferably, GLP-1 has a sequence of: 
6 10 20 30 37
XHXEGTFTSD VSSYLEXQAA XEFIAWLVXG XX
wherein the position 6 is: R or a deletion;
wherein the position 8 is: A, G or V;
the position 22 is: G or E;
the position 26 is: K, R, Q or N;
the position 34 is: K, R, Q or N;
the position 36 is: R, R-NH₂, K or K-NH₂; and
the position 37 is: G or a deletion.

As is well known, the insulinotropic peptide has poor stability, and it is very difficult for the aqueous solution thereof to get through its storage duration (2 years at 2 to 8°C). However, the present invention has developed a pharmaceutical composition capable of allowing the insulinotropic peptide, the analogue and the derivative to be stable, and allow them to meet the requirement for the storage duration. For example, in the formulation of the present invention, the GLP-1 concentration is up to 2 mg/ml, and in the event that an preservative is added, it can be stored for 2 years at 4°C.

Concentration is a factor that influences stability. If a drug molecule has good stability, its steady concentration is high, and on the contrary, its steady concentration is low. For a certain particular drug molecule, the drug has greater stability at a low concentration than that at a higher concentration.

In the pharmaceutical composition of the present invention, the insulinotropic peptide has a concentration of about 0.1 to 20 mg/mL, more preferably about 0.2 to 10 mg/mL, more preferably about 0.05 to 0.5 mg/mL, more preferably about 1 to 5 mg/mL, and more preferably about 2 to 4 mg/mL. In the present application, "about" refers to the difference from a stated numerical value in a range of ±10%.

Another factor that that plays an important role in stability is maintenance of the pH value of the pharmaceutical composition, and in particular, it is found in the present invention that maintenance of the pH value at about 3.5 to 4.0 is very good, and GLP-1 can maintain good stability within this range. Also, it is found that the pH value at which stability is kept has a very narrow range. The drug is very unstable at a pH value between about 4.5 and 6.5, and turbidity or precipitation will be occurred so long as the GLP-1 drug molecules are shifted into this pH range. When the pH value is lower than about 3.5, acid hydrolysis will be occurred, which is unstable likewise. Also, the injection formulation requires that the pH value should not be lower than about 3.0, most preferably not be lower than about 4.0, and a pH value lower than about 3.5 or lower is unfavourable for animal and human bodies.

The pharmaceutical composition of the present invention has a pH value of about 3.5 to 5.0, more preferably about 3.5 to 4.5, more preferably about 3.6 to 4.2, more preferably about 3.6 to 4.0, and more preferably about 3.6 to 3.9. At this pH, stability of GLP-1 comes up to what is expected, and can be stored for 2 years or more at 2 to 8°C.

In the process of formulating the GLP-1 pharmaceutical composition formulation, it is generally required to add a buffer salt to maintain pH of the pharmaceutical composition. In addition, the kind of the buffer will also influence the stability of GLP-1. Phosphate has poor stability, and the buffer salt should be able to provide a buffer salt within this pH range, hence histidine - HCl, sodium acetate - acetic acid, glycine - HCl, disodium hydrogen phosphate - citric acid, sodium hydroxide - citric acid, sodium citrate - citric acid, succinate - succinic acid, lactate - lactic acid, glutaminate - glutamic acid, malate - malic acid, benzoate - benzoic acid, tartrate - tartaric acid and the like may be employed. This buffer salt must be a pharmaceutically acceptable buffer salt, i.e., it has no adverse effect on GLP-1, and has pharmacology and toxicology that meet the requirements. The buffer salt is preferably histidine and sodium acetate - acetic acid, and most preferably sodium acetate - acetic acid.

Concentration of the buffer salt has a very great influence on the GLP-1 polypeptide. GLP-1 is highly sensible to the salt and is extremely unstable to the salt at a high concentration. Concentration of the buffer salt selected in the present invention is about 2 to 200 mmol/L, more preferably about 5 to 200 mmol/L, more preferably about 5 to 50 mmol/L, more preferably about 5 to 20 mmol/L, and most preferably about 7.5 to 10 mmol/L.

For a multi-dose parenteral solution, the preservative is an essential ingredient of the pharmaceutical composition formulation. Preservative refers to a natural or synthetic chemical ingredient, for adding into food, drugs, pigments, biological specimens and the like, to delay decomposition caused by microbial growth or chemical changes, and thereby to prolong shelf life of the food, drugs, pigments, biological specimens and the like. If no preservative is added into the polypeptide drug, it is extremely difficult to meet the quality control requirements of microbes for multiple administrations. Usage of the preservative has much to do with the kind of the preservative, the pH value of the pharmaceutical composition, the packaging material and the sealing material. Preservatives of the types such as Nipagin and benzoic acid have high preservative efficacy at an acidic condition, and reduced efficacy at an alkaline condition. Various preservatives all have effective antimicrobial concentrations thereof, and the concentration in use should not be lower than these concentrations. Also, the preservative should be used in an amount that is not too high, to prevent from doing harm to human bodies. Preservatives that may be used in drugs may influence stability of the polypeptide. Generally, phenols are used in the pharmaceutical composition as preservatives, but phenol preservatives have severe influences on the stability of polypeptides, and GLP-1 as well. Therefore, it is a difficult problem that must be solved to carefully select an preservative for a GLP-1 drug solution, which not only has an preservative effect, but also will not notably influence the stability of GLP-1, i.e., after employment, it allows the pharmaceutical composition to be able to meet the requirements for storage duration. The present invention has successfully solved this difficult problem.

When the insulinotropic peptide is GLP-1, the preservative may be phenol, benzyl alcohol, methyl p-hydroxybenzoate, ethyl p-hydroxybenzoate, propyl p-hydroxybenzoate, butyl p-hydroxybenzoate, chlorobutanol, 2-phenoxyethanol, 2-phenethyl alcohol, benzalkonium chloride (bromide), merthiolate or any combinations thereof. For a GLP-1 pharmaceutical composition, preferably benzyl alcohol, methyl p-hydroxybenzoate, ethyl p-hydroxybenzoate, propyl p-hydroxybenzoate and phenol are used, more preferably benzyl alcohol, phenol, or two of the above are used in combination.

Concentration of the preservative is also a factor to be taken into consideration. Different kinds of preservatives may have different antimicrobial concentrations in use. If metacresol or phenol is selected, the concentration in use is about 1 mg/mL to 10 mg/mL, more preferably about 1 mg/mL to 5 mg/mL, and most preferably about 1.5 mg/mL to 3 mg/mL. If benzyl alcohol is selected, the concentration in use is about 5 mg/mL to 20 mg/mL, more preferably about 5 mg/mL to 10 mg/mL, and most preferably about 7.5 mg/mL to 10 mg/mL.

In the formulation of the pharmaceutical composition, an isotonic agent should be selected carefully to allow the pharmaceutical composition to have an tonicity that is close to the human tonicity. In addition, many isotonic agents function as stabilizers at the same time. Not only an intrinsic tonicity of the isotonic agent, but also influences of other ingredients in the pharmaceutical composition on the overall tonicity of the composition should be taken into consideration in the selection of concentration of the isotonic agent. Isotonic agents employed in the present invention include polyols, for example, mannitol, sorbitol, inositol, xylitol, glycerin, propylene glycol and the like; sodium chloride; sugar, sucrose, trehalose, lactose, fructose and glucose and the like, and mannitol, glycerin and sorbitol are used preferentially, most preferably mannitol.

When polyol or sugar is used as the isotonic agent, it is used at a concentration of about 10 mg/mL to 100 mg/mL, and more preferably about 30 mg/mL to 50 mg/mL. When sodium chloride is used as the isotonic agent, it is used at a concentration of about 1 to 30 mg/mL, more preferably about 1 to 15 mg/mL, more preferably about 5 mg/mL to 15 mg/mL, and more preferably about 7 mg/mL to 9 mg/mL.

Because GLP-1 has strong hydrophobicity and is susceptible to self-association into macromolecular aggregates or generation of gels, a dissolution enhancer has a very good effect on the dissolution of GLP-1. Dissolution enhancers employed in the present invention include Tween 20, Tween 40, Tween 80, Span 20, Span 40, Span 80, Poloxamer 188, Pluronic F68, Brij 35, dextran, PEG 400, PEG 1000, PEG 1500, PEG 2000, propylene glycol, and the like, and propylene glycol and PEG 400 are used preferentially, most preferably propylene glycol. The dissolution enhancer is used at a concentration of about 0.1 mg/mL to 10 mg/mL, and preferably about 0.2 mg/mL to 5 mg/mL.

In another aspect, the present invention further provides use of the aqueous parenteral pharmaceutical composition formulated in the present invention, in particular, use thereof in the preparation of drugs for treating diabetes and adiposis.

The aqueous parenteral pharmaceutical composition of the present invention overcomes the problem with a parenteral solution in the prior art of difficulty in meeting the requirements for shelf life after the addition of an preservative, and is still able to be stored for 2 years at 4°C in the event that an preservative is added therein at a concentration up to 2 mg/ml.

The present invention is further set forth below in conjunction with particular embodiments. However, the present invention is not limited to these particular embodiments or examples.

### Detailed Description of the Preferred Embodiments

### Example 1 (reference example) Influence of pH value and ionic strength on stability of GLP-1

GLP-1 lyophilized powders were taken, dissolved with a 0.01 M sodium acetate - acetic acid buffer to 10 mg/mL, and then GLP-1 was replaced into buffers at various pH values through dialysis or through a G25 chromatographic method. Each buffer was also designed to have 4 salt concentrations. Samples collected were quantified by a HPLC method, and then GLP-1 concentration was adjusted to 4 mg/mL, followed by addition of an adjuvant to a required concentration. The final concentration of GLP-1 was 2 mg/mL. The kinds of the buffers and designs of pharmaceutical compositions are as shown in Table 1.

**Table 1. Observation of influences of pH and salt concentration on physical stability of GLP-1**

| Type of the buffers | pH | Replacement methods of the buffers | NaCl concentration (mmol/L) | Situations of samples after preparation |
|---|---|---|---|---|
| 20 mmol/L NaAC-HAC | 3.0 | Sephadex G25 | 0 | Clear and transparent |
| | | | 20 | Clear and transparent |
| | | | 150 | Clear and transparent |
| | | | 500 | Clear and transparent |
| | 3.5 | Sephadex G25 | 0 | Clear and transparent |
| | | | 20 | Clear and transparent |
| | | | 150 | Clear and transparent |
| | | | 500 | Turbid |
| | 4.0 | Sephadex G25 | 0 | Clear and transparent |
| | | | 20 | Clear and transparent |
| | | | 150 | Clear and transparent |
| | | | 500 | Turbid |
| | 4.5 | Dialysis | 0 | Difficult to filter |
| | | | 20 | Difficult to filter |
| | | | 150 | Difficult to filter |
| | | | 500 | Difficult to filter, turbid |
| | 5.0 | Dialysis | 0 | Turbid or precipitated |
| | | | 20 | |
| | | | 150 | |
| | | | 500 | |
| 20 mmol/L | 5.5 | Dialysis | 0 | Turbid or precipitated |
| citrate sodium - citric acid | | | 20 | |
| | | | 150 | |
| | | | 500 | |
| | 6.0 | Sephadex G25 | 0 | Precipitate sticking to the wall |
| | | | 20 | Precipitate sticking to the wall |
| | | | 150 | Precipitate sticking to the wall |
| | | | 500 | Precipitate sticking to the wall |
| | 6.5 | Sephadex G25 | 0 | Turbid |
| | | | 20 | Turbid |
| | | | 150 | Turbid |
| | | | 500 | Turbid |
| 20 mmol/L Na₂HPO₄ - NaH₂PO₄ | 7.0 | Sephadex G25 | 0 | Clear and transparent |
| | | | 20 | Clear and transparent |
| | | | 150 | Clear and transparent |
| | | | 500 | Clear and transparent |
| | 7.5 | Sephadex G25 | 0 | Clear and transparent |
| | | | 20 | Clear and transparent |
| | | | 150 | Clear and transparent |
| | | | 500 | Clear and transparent |

Samples were placed at two temperatures, i.e., 25°C and 35°C, for treatment, and taken out for detection at 8 days.

An investigation method for physical stability: before determination, the samples were observed for appearance with the naked eye. If there was evident precipitation or turbidity, no further determination would be carried out. If the samples had no abnormal phenomena observable with the naked eye, the samples were taken and subjected to determination of absorption values at 360 nm and HPLC detection, wherein the former investigated the physical stability, and the latter investigated the chemical stability.

Absorption values determined at a wavelength of 360 nm were compared, so as to compare differences in physical stability among the samples. A higher absorption value indicated poorer physical stability.

An investigation method for chemical stability: a test sample was taken, and analyzed on a C18 column (3.5 µm, 300 Å, Φ 4.6 x 50 mm). An analytical method: mobile phase A was 0.1% trifluoroacetic acid, B was the A phase with 80% acetonitrile added therein, the detection wavelength was 280 nm, 60% A and 40% B were balanced for 3 min, and the sample amount was 2 to 5 µl. Elution was performed for 8 min with 40% to 53% of the B phase and 60% to 47% of the A phase. Peak area was calculated using the normalization method. A standard sample was determined with the same method. The peptide concentration in the sample was calculated by comparison with the standard sample, and compared with the determination result at day 0, to calculate the retention of the peptide content in the sample. A higher retention of the peptide content indicated better chemical stability.

Stability results of GLP-1 in solutions at various pH values are as follows.

**Table 2. Influences of pH and salt concentration on stability of GLP-1**

| pH | NaCl concentration (mg/mL) | Absorption values at 360 nm | | Results of HPLC analysis, retention of the peptide content (%) | |
|---|---|---|---|---|---|
| | | 35°C, 8 days | 45°C, 8 days | 35°C, 8 days | 45°C, 8 days |
| 3.0 | 0 | 0.0706 | 0.0844 | 95.46 | 87.19 |
| | 1.17 | 0.0716 | 0.0724 | 93.69 | 87.93 |
| | 8.77 | 0.1697 | 0.1812 | 95.03 | 87.86 |
| | 29.22 | 0.2052 | 0.2417 | 96.42 | 91.16 |
| 3.5 | 0 | 0.0684 | 0.0689 | 94.73 | 88.66 |
| | 1.17 | 0.0694 | 0.0823 | 96.92 | 92.54 |
| | 8.77 | 0.1882 | 0.1780 | 98.06 | 92.90 |
| | 29.22 | Samples became turbid by placement for a period of time after formulation. | | | |
| 4.0 | 0 | 0.0686 | 0.0843 | 96.07 | 92.77 |
| | 1.17 | 0.1354 | 0.1448 | 97.43 | 93.76 |
| | 8.77 | 0.1907 | 0.1974 | 98.73 | Turbid |
| | 29.22 | Samples became turbid after formulation. | | | |
| 4.5 | 0 | 0.1326 | 0.1370 | 97.23 | 92.45 |
| | 1.17 | 0.1655 | 0.1814 | 95.29 | Turbid |
| | 8.77 | 1.4096 | 1.3635 | Turbid | Turbid |
| | 29.22 | Samples became turbid after formulation. | | | |
| 5.0 | 0 | Precipitate was generated while GLP-1 was prepared. | | | |
| | 1.17 | | | | |
| | 8.77 | | | | |
| | 29.22 | | | | |
| 5.5 | 0 | Precipitate was generated while GLP-1 was prepared. | | | |
| | 1.17 | | | | |
| | 8.77 | | | | |
| | 29.22 | | | | |
| 6.0 | 0 | Precipitate was generated after formulation and placement of the sample. | | | |
| | 1.17 | | | | |
| | 8.77 | | | | |
| | 29.22 | | | | |
| 6.5 | 0 | Turbidity was generated after formulation and placement of the sample. | | | |
| | 1.17 | | | | |
| | 8.77 | | | | |
| | 29.22 | | | | |
| 7.0 | 0 | 0.0813 | 0.0828 | 94.30 | 88.83 |
| | 1.17 | 0.0864 | 0.0865 | 93.35 | 90.43 |
| | 8.77 | 0.0824 | 0.0841 | 94.34 | 92.79 |
| | 29.22 | 0.0850 | 0.0862 | 95.83 | 95.37 |
| 7.5 | 0 | 0.0807 | 0.0859 | 90.89 | |
| | 1.17 | 0.0799 | 0.0937 | 91.88 | 85.88 |
| | 8.77 | 0.0846 | 0.0902 | 92.15 | 87.48 |
| | 29.22 | 0.0861 | 0.0942 | 93.74 | 88.04 |

| | | | | | |
|---|---|---|---|---|---|
| Note: an absorption value of the control background colour was 0 to 0.1, and opalescence or turbidity could be seen with the naked eye when it was greater than 0.12. | | | | | |

Conclusions: when GLP-1 was at pH 5.0 to 6.5, turbidity or precipitation was occurred while the GLP-1 sample was prepared, indicating that GLP-1 is unstable at these pH values; whereas when pH was 4.5, the sample prepared was clear and transparent, but turbidity was occurred in case of high salt concentration when formulated as a pharmaceutical composition and then subjected to heating treatment. At pH 3.5 and pH 4.0, turbidity was occurred in the sample in the presence of 0.5 M NaCl, and no turbidity or precipitation was seen only at pH 3.0. As can also be seen from the absorption values determined at 360 nm, the absorption value was increased with the increasing pH value between pH 3.0 and 4.5, indicating that the physical stability was decreased. At the same pH value, the absorption value was also increased with the increasing NaCl salt concentration, indicating that the salt decreased the physical stability. However, the HPLC analysis was then contrary, the retention of the peptide content was increased with the increasing pH value, indicating that a superacidic condition is unfavourable for the chemical stability of GLP-1. Whereas at the same pH value, the retention of the peptide content was increased with the increasing salt concentration, possibly due to a certain inhibitory effect of the salt on GLP-1 adsorption. At pH 7.0 and 7.5, the physical stability was all normal, but the retention of the peptide content was significantly lower as compared with the samples at pH 3.0 to 4.5, and therefore the chemical stability was poor at pH 7.0 to 7.5.

The following table shows the physical stability and chemical stability of the pharmaceutical composition when pH of the GLP-1 pharmaceutical composition ranged from 3.6 to 4.2 and the sodium chloride salt concentration was below 20 mmol/L (1.17 mg/mL). A monitoring method for physical stability of the pH value was the fluorescence value, that is, thioflavine T was added into the sample at a final concentration of 5 µmol/L, then the fluorescence absorption value was determined (at an excitation wavelength of 435 nm, and an emission wavelength of 485 nm). The higher the absorption value is, the severer the gelatination phenomena of the sample is, and the poorer the physical stability is.

**Table 3. Influence of the pH value on stability of GLP-1**

| pH | Results of HPLC analysis, retention of the peptide content % | | Fluorescence value | |
|---|---|---|---|---|
| | 25°C, 30 days | 35°C, 30 days | 25°C, 35 days | 35°C, 35 days |
| 3.6 | 93.04 | 86.25 | 8.49 | 9.95 |
| 3.7 | 95.15 | 86.11 | 9.11 | 14.55 |
| 3.8 | 97.10 | 89.48 | 11.01 | 31.58 |
| 3.9 | 98.08 | 90.36 | 26.46 | 78.27 |
| 4.0 | 97.85 | 91.80 | 59.32 | 113.18 |
| 4.1 | 98.90 | 91.35 | 139.48 | 157.00 |
| 4.2 | 98.24 | 93.96 | N.D | N.D |

As can be seen from the results in Table 3, between a range from pH 3.6 to 4.2, the lower the pH value is, the better the physical stability is, and the poorer the chemical stability is, and on the contrary, the higher the pH value is, the better the chemical stability is, and the poorer the physical stability is. Therefore, the pH range at which GLP-1 is stable is supposed to be a result of comprehensive consideration of both physical and chemical stability.

### Example 2 (reference example) Preparation of the formulation pharmaceutical composition

20 mL of 4 mg/mL GLP-1 peptide (in a 20 mmol/L buffer, pH 3.5 to 4.5) was mixed with 20 mL of 80 mg/mL mannitol - 5.2 mg/mL phenol. The mixture was adjusted to pH 3.5-4.5 with NaOH or acetic acid, filtered through a 0.22 µm filter membrane, and dispensed into 2 mL Penicillin bottles. Each of the components was:

| | |
|---|---|
| GLP-1 | 2 mg/mL |
| Mannitol | 40 mg/mL |
| Phenol | 2.6 mg/mL |
| NaAC-HAC | 10 mmol/L |
| pH 3.5 to 4.5 | |

The samples dispensed were placed at 25°C and 35°C respectively. Samples were taken at different times for inspection and analysis, to investigate physical and chemical stability.

### Example 3 (reference example) Influences of buffer systems and antimicrobial agents on the physical stability of GLP-1

The GLP-1 solution (referred to as a stock solution) that had been replaced into different buffer systems (the buffers had a concentration 2 times that of the final pharmaceutical composition) was diluted with a buffer to 4 mg/mL, and an equal volume of a concentrated stock adjuvant solution with a 2-time final concentration was added therein. The solutions were mixed uniformly, filtered through a 0.22 µm filter membrane, dispensed into 2 mL Penicillin bottles, and placed at different temperatures for investigation. A series of sampling time points were arranged. After sampling, the samples were firstly observed with the naked eye for appearance. If evident turbidity or precipitation was occurred, the sample was considered as disqualified as for physical stability, and would not be subjected to the next step of HPLC analysis.

Designs and results are as shown in Table 4:

**Table 4. Influence of composition of the pharmaceutical composition on the physical stability of GLP-1**

| Pharmaceutical composition No. | Composition (GLP-1 final concentration 2 mg/mL) | Appearance after preparation | 25°C, 14 days |
|---|---|---|---|
| 1 | 10 mmol/L NaAC-HAC pH 3.5, 40 mg/mL mannitol, 3 mg/mL metacresol | Turbid | Turbid |
| 2 | 10 mmol/L NaAC-HAC pH 3.5, 45 mg/mL mannitol, 3 mg/mL metacresol | Turbid | Turbid |
| 3 | 10 mmol/L NaAC-HAC pH 3.5, 50 mg/mL mannitol, 3 mg/mL metacresol | Turbid | Turbid |
| 4 | 10 mmol/L NaAC-HAC pH 3.5, 55 mg/mL mannitol, 3 mg/mL metacresol | Turbid | Turbid |
| 5 | 10 mmol/L NaAC-HAC pH 3.5, 40 mg/mL mannitol, 2 mg/mL phenol | Transparent and clear | Transparent and clear |
| 6 | 10 mmol/L NaAC-HAC pH 3.5, 45 mg/mL mannitol, 2 mg/mL phenol | Transparent and clear | Transparent and clear |
| 7 | 10 mmol/L NaAC-HAC pH 3.5, 50 mg/mL mannitol, 2 mg/mL phenol | Transparent and clear | Transparent and clear |
| 8 | 10 mmol/L NaAC-HAC pH 3.5, 55 mg/mL mannitol, 2 mg/mL phenol | Transparent and clear | Transparent and clear |
| 9 | 10 mmol/L NaAC-HAC pH 3.5, 10 mg/mL glycerin, 3 mg/mL metacresol | Turbid | Turbid |
| 10 | 10 mmol/L NaAC-HAC pH 3.5, 1.50 mg/mL glycerin, 3 mg/mL metacresol | Turbid | Turbid |
| 11 | 10 mmol/L NaAC-HAC pH 3.5, 20 mg/mL glycerin, 3 mg/mL metacresol | Turbid | Turbid |
| 12 | 10 mmol/L NaAC-HAC pH 3.5, 25 mg/mL glycerin, 3 mg/mL metacresol | Turbid | Turbid |
| 13 | 10 mmol/L histidine pH 4.0, 40 mg/mL mannitol, 2 mg/mL phenol | Transparent and clear | Transparent and clear |
| 14 | 10 mmol/L histidine pH 4.0, 45 mg/mL mannitol, 2 mg/mL phenol | Transparent and clear | Transparent and clear |
| 15 | 10 mmol/L histidine pH 4.0, 50 mg/mL mannitol, 2 mg/mL phenol | Transparent and clear | Transparent and clear |
| 16 | 10 mmol/L histidine pH 4.0, 55 mg/mL mannitol, 2 mg/mL phenol | Transparent and clear | Transparent and clear |
| 17 | 10 mmol/L histidine pH 4.0, 15 mg/mL glycerin, 2 mg/mL phenol | Transparent and clear | Transparent and clear |
| 18 | 10 mmol/L histidine pH 4.0, 20 mg/mL glycerin, 2 mg/mL phenol | Transparent and clear | Transparent and clear |
| 19 | 10 mmol/L histidine pH 4.0, 25 mg/mL glycerin, 2 mg/mL phenol | Transparent and clear | Transparent and clear |
| 20 | 10 mmol/L NaAC-HAC pH 3.5, 40 mg/mL mannitol, 9 mg/mL benzyl alcohol | Transparent and clear | Transparent and clear |
| 21 | 10 mmol/L NaAC-HAC pH 3.5, 45 mg/mL mannitol, 9 mg/mL benzyl alcohol | Transparent and clear | Transparent and clear |
| 22 | 10 mmol/L NaAC-HAC pH 3.5, 50 mg/mL mannitol, 9 mg/mL benzyl alcohol | Transparent and clear | Transparent and clear |

As can be seen from the results in Table 4, metacresol severely influenced the stability of GLP-1, which became turbid immediately after the formulation, whereas phenol and benzyl alcohol were better, as the pharmaceutical composition solutions of GLP-1 maintained in a clear and transparent state.

### Example 4 (reference example) Influence of the adjuvant on physical stability of GLP-1

**Table 5. Influence of the adjuvant on physical stability of GLP-1**

| Pharmaceutical composition No. | Composition (GLP-1 final concentration 2 mg/mL, 10 mmol/L NaAC-HAC pH 3.5) | Appearance after preparation | 25°C, 14 days |
|---|---|---|---|
| 23 | 40 mg/mL sorbitol, 2 mg/mL phenol | Transparent and clear | Transparent and clear |
| 24 | 45 mg/mL sorbitol, 2 mg/mL phenol | Transparent and clear | Transparent and clear |
| 25 | 50 mg/mL sorbitol, 2 mg/mL phenol | Transparent and clear | Transparent and clear |
| 26 | 5 mg/mL hydroxypropyl-beta-cyclodextrin, 2 mg/mL phenol | Transparent and clear | Transparent and clear |
| 27 | 20 mg/mL hydroxypropyl-beta-cyclodextrin, 2 mg/mL phenol | Transparent and clear | Transparent and clear |
| 28 | 5 mg/mL hydroxypropyl-beta-cyclodextrin (HP), 3 mg/mL metacresol | Turbid | Turbid |
| 29 | 20 mg/mL hydroxypropyl-beta-cyclodextrin, 3 mg/mL metacresol | Turbid | Turbid |
| 30 | 5 mg/mL hydroxypropyl-beta-cyclodextrin, 9 mg/mL benzyl alcohol | Transparent and clear | Transparent and clear |
| 31 | 20 mg/mL hydroxypropyl-beta-cyclodextrin, 9 mg/mL benzyl alcohol | Transparent and clear | Transparent and clear |
| 32 | 0.5 mg/mL carboxyl methyl cellulose (CMC), 2 mg/mL phenol | Turbid | Turbid |
| 33 | 0.5 mg/mL carboxyl methyl cellulose (CMC), 3 mg/mL metacresol | Turbid | Turbid |
| 34 | 0.5 mg/mL carboxyl methyl cellulose (CMC), 9 mg/mL benzyl alcohol | Turbid | Turbid |
| 35 | 2 mg/mL carboxyl methyl cellulose (CMC), 2 mg/mL phenol | Turbid | Turbid |
| 36 | 2 mg/mL carboxyl methyl cellulose (CMC), 3 mg/mL metacresol | Turbid | Turbid |
| 37 | 2 mg/mL carboxyl methyl cellulose (CMC), 9 mg/mL benzyl alcohol | Turbid | Turbid |
| 38 | 0.05 mg/mL heparin sodium, 2 mg/mL phenol | Turbid | Turbid |
| 39 | 0.05 mg/mL heparin sodium, 3 mg/mL metacresol | Turbid | Turbid |
| 40 | 0.05 mg/mL heparin sodium, 9 mg/mL benzyl alcohol | Turbid | Turbid |
| 41 | 0.3 mg/mL heparin sodium, 2 mg/mL phenol | Turbid | Turbid |
| 42 | 0.3 mg/mL heparin sodium, 3 mg/mL metacresol | Turbid | Turbid |
| 43 | 0.3 mg/mL heparin sodium, 9 mg/mL benzyl alcohol | Turbid | Turbid |

Results in Table 5 show that carboxy methylcellulose and heparin sodium are not suitable as adjuvants of GLP-1.

### Example 5 (reference example) Influence of the adjuvant (additive) on the stability of GLP-1

Formulation of the pharmaceutical composition:
The GLP-1 solution (referred to as a stock solution) that had been replaced into different buffer systems (the buffers had a concentration 2 times that of the final pharmaceutical composition) was diluted with a buffer to 4 mg/mL, and an equal volume of a concentrated stock adjuvant solution with a 2-time concentration was added therein. The solutions were adjusted to pH 3.5-4.0, mixed uniformly, filtered through a 0.22 µm filter membrane, dispensed into 2 mL Penicillin bottles, and placed at different temperatures for investigation. A series of sampling time points were arranged. The samples were firstly observed with the naked eye for appearance. If no evident turbidity or gelatination was occurred, the sample was considered as qualified as for physical stability, and then subjected to HPLC detection to analyze the chemical stability.

The HPLC detection method was performed according to Example 1.

**Table 6. Influence of composition of the pharmaceutical composition on the stability of GLP-1**

| Pharmaceuti cal composition No. | Composition (GLP-1 final concentration 2 mg/mL) | | Results of HPLC analysis 0 day after the preparation | | 25°C, 14 days | |
|---|---|---|---|---|---|---|
| | | | Purity (%) | Peptide concentrati on (mg/ml) | Purity (%) | Retentio n of the peptide content (%) |
| 44 | 10 mmol/L histidine - HCl pH 4.0 | 45 mg/ml mannitol, 2 mg/ml phenol, 0.1 mg/ml Tween 80 | 97.97 | 1.96 | Gelatination | |
| 45 | | 45 mg/ml mannitol, 2 mg/ml phenol, 0.5 mg/ml Tween 80 | 97.78 | 2.00 | 97.12 | 91.51 |
| 46 | | 45 mg/ml mannitol, 2% phenol, 2 mg/ml Tween 80 | 97.62 | 2.00 | 97.72 | 86.55 |
| 47 | 10 mmol/L NaAC - HAC, pH 3.5 | 45 mg/ml mannitol, 2 mg/ml phenol, 0.1 mg/ml Tween 80 | 98.24 | 1.97 | 97.99 | 92.39 |
| 48 | | 45 mg/ml mannitol, 2 mg/ml phenol, 0.5 mg/ml Tween 80 | 98.35 | 1.96 | 98.42 | 92.21 |
| 49 | | 45 mg/ml mannitol, 2 mg/ml phenol, 2 mg/ml Tween 80 | 98.37 | 2.01 | 97.57 | 91.86 |
| 50 | 10 mmol/L NaA - HAC, pH 4.0 | 45 mg/ml mannitol, 2 mg/ml phenol, 0.2 mg/ml PEG 400 | 97.88 | 2.01 | 97.72 | 80.37 |
| 51 | | 45 mg/ml mannitol, 2 mg/ml phenol, 0.5 mg/ml PEG 400 | 97.78 | 2.03 | 97.79 | 93.55 |
| 52 | | 45 mg/ml mannitol, 2 mg/ml phenol, 1 mg/ml PEG 400 | 97.59 | 2.00 | 97.74 | 89.60 |
| 53 | 10 mmol/L histidine - | 45 mg/ml mannitol, 2 mg/ml phenol, 0.2 mg/ml PEG 400 | 98.28 | 2.06 | 97.19 | 90.07 |
| 54 | HCl pH 4.0 | 45 mg/ml mannitol, 2 mg/ml phenol, 0.5% PEG 400 | 98.48 | 2.06 | 97.74 | 90.30 |
| 55 | | 45 mg/ml mannitol, 2 mg/ml phenol, 1 mg/ml PEG 400 | 98.42 | 1.85 | 97.39 | 87.39 |

All pharmaceutical compositions of 10 mmol/L histidine pH 4.0 were placed at 4°C overnight, and then milkiness appeared. When the pharmaceutical compositions were shifted to room temperature (above 25°C), they became clear.

### Example 6

The formulation method and HPLC detection method of the pharmaceutical compositions were performed according to Example 5.

**Table 7. Influence of composition of the pharmaceutical composition on the stability of GLP-1. Compositions 56-59 are not according to the invention.**

| Pharmaceutic al composition No. | Composition (GLP-1 final concentration 2 mg/mL, 10 mmol/L NaAC-HAC pH 3.5) | Results of HPLC analysis (0 day) after the preparation | | 25°C, 42 days | |
|---|---|---|---|---|---|
| | | Purity (%) | Peptide concentrati on (mg/mL) | Purity (%) | Retention of the peptide content (%) |
| 56 | 50 mg/mL mannitol + 2 mg/mL phenol + 0.5 mg/mL dextran 20 | 99.5 | 2.04 | Gelatination | |
| 57 | 50 mg/mL mannitol + 2 mg/mL phenol + 1 mg/mL dextran 20 | 100 | 2.01 | Gelatination | |
| 58 | 50 mg/mL mannitol + 2 mg/mL phenol + 2 mg/mL dextran 20 | 99.5 | 2.14 | Gelatination | |
| 59 | 50 mg/mL mannitol + 2 mg/mL + 3 mg/mL dextran 20 | 99.6 | 2.11 | Gelatination | |
| 60 | 50 mg/mL mannitol + 2 mg/mL phenol + 0.1 mg/mL propylene glycol | 99.4 | 1.95 | 97.8 | 98.5 |
| 61 | 50 mg/mL mannitol + 2 mg/mL phenol + 0.4 mg/mL propylene glycol | 99.7 | 1.95 | 97.8 | 98.37 |
| 62 | 50 mg/mL mannitol + 2 mg/mL phenol + 1 mg/mL propylene glycol | 100 | 2.00 | 97.9 | 97.69 |

### Example 7

The formulation method and HPLC detection method of the pharmaceutical compositions were performed according to Example 5.

**Table 8. Influence of the ratio of mannitol to propylene glycol on the stability of GLP-1. Composition 63 is not according to the invention.**

| Pharmaceutic al composition No. | Composition (GLP-1 final concentration 2 mg/mL, 10 mmol/L NaAC-HAC pH 3.5) | Results of HPLC analysis (0 day) after the preparation | | 25°C, 42 days | |
|---|---|---|---|---|---|
| | | Purity (%) | Peptide concentrati on (mg/mL) | Purity (%) | Retention of the peptide content (%) |
| 63 | 40 mg/mL mannitol + 2.2 mg/mL phenol | 99.7 | 2.01 | 97.0 | 96.65 |
| 64 | 40 mg/mL mannitol + 2.2 mg/mL phenol + 0.1 mg/mL propylene glycol | 99.4 | 1.99 | 97.8 | 97.62 |
| 65 | 38 mg/mL mannitol + 2.2 mg/mL phenol + 1 mg/mL propylene glycol | 99.4 | 1.99 | 97.8 | 96.66 |
| 66 | 35 mg/mL mannitol + 2.2 mg/mL phenol + 2 mg/mL propylene glycol | 99.5 | 2.00 | 97.5 | 97.93 |
| 67 | 30 mg/mL mannitol + 2.2 mg/mL phenol + 5 mg/mL propylene glycol | 99.5 | 2.00 | 96.7 | 97.19 |
| 68 | 22 mg/mL mannitol + 2.2 mg/mL phenol + 7.5 mg/mL propylene glycol | 99.5 | 2.00 | 97.7 | 97.07 |

As can be seen from results in Table 8, two pharmaceutical compositions, i.e., 3.5% mannitol + 0.2% propylene glycol and 4% mannitol + 0.01% propylene glycol, had the highest retention of the peptide content at 25 degrees at 42 days.

### Example 8 (not according to the invention)

An analogue Em of Exendin-4 had a sequence as follows:
HGEGTFTSDL SKQLEEEAVK LFIEWLKNGG PSSGAPPPR

### (1) Preparation of Em

The preparation was performed using a solid phase polypeptide synthesis method, and then purification using a reversed phase C18 column and lyophilization were performed, so as to obtain Em.

### (2) Formulation method of the pharmaceutical composition

Lyophilized Em powder was weighed, and dissolved with 2 times of a pH 3.5 NaAC-HAC buffer. In addition, mannitol crystalline powder and metacresol were weighed according to an amount 2 times that in recipe, and dissolved in water. Then, the above two solutions were mixed, stirred uniformly, filtered through a 0.22 µm membrane, and dispensed into Penicillin bottles or carlsberg's flasks.

### (3) HPLC detection method

An investigation method for chemical stability: a test sample was taken, and analyzed on a C18 column (5.0 µm, 300 Å, Φ 4.6 x 150 mm). An analytical method: mobile phase A was 0.1% trifluoroacetic acid, B was the A phase with 80% acetonitrile added therein, the detection wavelength was 214 nm, 68% A and 32% B were balanced for 4 min, and the sample amount was 20 to 40 µl. Elution was performed for 15 min with 32% to 45% of the B phase and 68% to 55% of the A phase. Peak area was calculated using the normalization method. A standard sample was determined with the same method. The peptide concentration in the sample was calculated by comparison with the standard sample, and compared with the determination result at day 0, to calculate the retention of the peptide content in the sample. A higher retention of the peptide content indicated better chemical stability.

**Table 9. Influence of composition of the composition on the stability of EM**

| Pharmaceutic al composition No. | Composition (EM final concentration 0.3 mg/mL, 10 mmol/L NaAC-HAC pH 3.5) | Results of HPLC analysis (0 day) after the preparation | | 25°C, 42 days | |
|---|---|---|---|---|---|
| | | Purity (%) | Peptide concentrati on (mg/mL) | Purity (%) | Retentio n of the peptide content (%) |
| 69 | 50 mg/mL mannitol + 0.3 mg/mLEDTA - Na₂ + 2.2 mg/mL metacresol | 97.6 | 0.296 | 92.78 | 93.40 |
| 70 | 50 mg/mL mannitol + 0.2 mg/mL Tween 80 + 2.2 mg/mL metacresol | 97.80 | 0.281 | 93.64 | 96.3 |
| 71 | 50 mg/mL mannitol + 2.2 mg/mL metacresol | 96.33 | 0.289 | 96.05 | 97.37 |
| 72 | 50 mg/mL mannitol + 0.3 mg/mL EDTA + 2.2 mg/mL metacresol + 0.2 mg/mL Tween 80 | 96.86 | 0.288 | 93.75 | 95.14 |

As can be seen from results in Table 9, the 50 mg/mL mannitol + 2.2 mg/mL metacresol formula had the highest retention of the peptide content as well as the best purity at 25 degrees at 30 days. That is to say, the recipe without the addition of EDTA or Tween 80 had good stability, and the recipe with the addition of EDTA or Tween 80 had poor stability.

## Claims

1. A stable insulinotropic peptide multi-dose injectable aqueous parenteral pharmaceutical composition that can be stored for a long term, the aqueous parenteral pharmaceutical composition comprising an insulinotropic peptide, mannitol, propylene glycol, phenol and a pharmaceutically acceptable buffer salt solution of sodium acetate-acetic acid (NaAc-HAc);
wherein, the aqueous parenteral pharmaceutical composition has a pH value of 3.0 to 5.0;
the insulinotropic peptide has a concentration of 0.1 mg/mL to 20 mg/mL, and preferably 0.5 mg/mL to 5 mg/mL; and
wherein the insulinotropic peptide is GLP-1.

2. The multi-dose injectable aqueous parenteral pharmaceutical composition of claim 1, wherein the aqueous parenteral pharmaceutical composition has a pH value of 3.5 to 4.5.

3. The multi-dose injectable aqueous parenteral pharmaceutical composition of claim 1, wherein the GLP-1 has a concentration of 1 mg/mL to 5 mg/mL, and preferably 2 mg/mL to 4 mg/mL.

4. The multi-dose injectable aqueous parenteral pharmaceutical composition of claim 1, wherein the mannitol has a concentration of 10 mg/mL to 100 mg/mL, and preferably 30 mg/mL to 50 mg/mL.

5. The multi-dose injectable aqueous parenteral pharmaceutical composition of claim 1, wherein the propylene glycol has a concentration of 0.01 mg/mL to 10 mg/mL, preferably 0.1 mg/mL to 10 mg/mL, more preferably 0.2 mg/mL to 2 mg/mL, and most preferably 0.5 mg/mL to 1 mg/mL.

6. The multi-dose injectable aqueous parenteral pharmaceutical composition of claim 1, wherein the concentration of phenol is 1 mg/mL to 20 mg/mL.

7. The multi-dose injectable aqueous parenteral pharmaceutical composition of claim 1, wherein the sodium acetate - acetic acid (NaAC-HAC) has a concentration of 2 to 200 mmol/L, preferably 5 to 50 mmol/L, more preferably 5 to 20 mmol/L, and most preferably 7.5 to 10 mmol/L.

8. The multi-dose injectable aqueous parenteral pharmaceutical composition of any one of claims 1 to 7 for use in treating diabetes.

9. The multi-dose injectable aqueous parenteral pharmaceutical composition of any one of claims 1 to 7 for use in treating adiposis.

## Patentansprüche

1. Stabile injizierbare wässrige parenterale pharmazeutische Mehrfachdosis-Zusammensetzung mit insulinotropem Peptid, die über eine lange Zeit gelagert werden kann, wobei die wässrige parenterale pharmazeutische Zusammensetzung ein insulinotropes Peptid, Mannitol, Propylenglykol, Phenol und eine pharmazeutisch akzeptable Puffersalzlösung aus Natriumacetat-Essigsäure (NaAc-HAc) umfasst;
wobei die wässrige parenterale pharmazeutische Zusammensetzung einen pH-Wert von 3,0 bis 5,0 aufweist; das insulinotrope Peptid eine Konzentration von 0,1 mg/ml bis 20 mg/ml und vorzugsweise 0,5 mg/ml bis 5 mg/ml aufweist; und
wobei das insulinotrope Peptid GLP-1 ist.

2. Injizierbare wässrige parenterale pharmazeutische Mehrfachdosis-Zusammensetzung nach Anspruch 1, wobei die wässrige parenterale pharmazeutische Zusammensetzung einen pH-Wert von 3,5 bis 4,5 aufweist.

3. Injizierbare wässrige parenterale pharmazeutische Mehrfachdosis-Zusammensetzung nach Anspruch 1, wobei das GLP-1 eine Konzentration von 1 mg/ml bis 5 mg/ml und vorzugsweise 2 mg/ml bis 4 mg/ml aufweist.

4. Injizierbare wässrige parenterale pharmazeutische Mehrfachdosis-Zusammensetzung nach Anspruch 1, wobei das Mannitol eine Konzentration von 10 mg/ml bis 100 mg/ml und vorzugsweise 30 mg/ml bis 50 mg/ml aufweist.

5. Injizierbare wässrige parenterale pharmazeutische Mehrfachdosis-Zusammensetzung nach Anspruch 1, wobei das Propylenglykol eine Konzentration von 0,01 mg/ml bis 10 mg/ml, bevorzugt 0,1 mg/ml bis 10 mg/ml, besonders bevorzugt 0,2 mg/ml bis 2 mg/ml und ganz besonders bevorzugt 0,5 mg/ml bis 1 mg/ml, aufweist.

6. Injizierbare wässrige parenterale pharmazeutische Mehrfachdosis-Zusammensetzung nach Anspruch 1, wobei die Konzentration von Phenol 1 mg/ml bis 20 mg/ml beträgt.

7. Injizierbare wässrige parenterale pharmazeutische Mehrfachdosis-Zusammensetzung nach Anspruch 1, wobei die Natriumacetat-Essigsäure (NaAc-HAc) eine Konzentration von 2 bis 200 mmol/l, bevorzugt 5 bis 50 mmol/l, besonders bevorzugt 5 bis 20 mmol/I und ganz besonders bevorzugt 7,5 bis 10 mmol/l, aufweist.

8. Injizierbare wässrige parenterale pharmazeutische Mehrfachdosis-Zusammensetzung nach einem der Ansprüche 1 bis 7 zur Verwendung bei der Behandlung von Diabetes.

9. Injizierbare wässrige parenterale pharmazeutische Mehrfachdosis-Zusammensetzung nach einem der Ansprüche 1 bis 7 zur Verwendung bei der Behandlung von Adipositas.

## Revendications

1. Composition pharmaceutique aqueuse parentérale injectable multidose stable de peptide insulinotrope qui peut être stockée à long terme, la composition pharmaceutique aqueuse parentérale comprenant un peptide insulinotrope, du mannitol, du propylène glycol, du phénol et une solution tampon de sel d'acétate de sodium-acide acétique (NaAc-HAc) pharmaceutiquement acceptable ;
la composition pharmaceutique aqueuse parentérale ayant un pH de 3,0 à 5,0 ;
le peptide insulinotrope ayant une concentration de 0,1 mg/ml à 20 mg/ml, et préférablement de 0,5 mg/ml à 5 mg/ml ; et
le peptide insulinotrope étant le GLP-1.

2. Composition pharmaceutique aqueuse parentérale injectable multidose selon la revendication 1, la composition pharmaceutique aqueuse parentérale ayant un pH de 3,5 à 4,5.

3. Composition pharmaceutique aqueuse parentérale injectable multidose selon la revendication 1, le GLP-1 ayant une concentration de 1 mg/ml à 5 mg/ml, et préférablement de 2 mg/ml à 4 mg/ml.

4. Composition pharmaceutique aqueuse parentérale injectable multidose selon la revendication 1, le mannitol ayant une concentration de 10 mg/ml à 100 mg/ml, et préférablement de 30 mg/ml à 50 mg/ml.

5. Composition pharmaceutique aqueuse parentérale injectable multidose selon la revendication 1, le propylène glycol ayant une concentration de 0,01 mg/ml à 10 mg/ml, préférablement de 0,1 mg/ml à 10 mg/ml, plus préférablement de 0,2 mg/ml à 2 mg/ml, et le plus préférablement de 0,5 mg/ml à 1 mg/ml.

6. Composition pharmaceutique aqueuse parentérale injectable multidose selon la revendication 1, la concentration de phénol étant de 1 mg/ml à 20 mg/ml.

7. Composition pharmaceutique aqueuse parentérale injectable multidose selon la revendication 1, l'acétate de sodium-acide acétique (NaAc-HAc) ayant une concentration de 2 à 200 mmol/l, préférablement de 5 à 50 mmol/l, plus préférablement de 5 à 20 mmol/l, et le plus préférablement de 7,5 à 10 mmol/l.

8. Composition pharmaceutique aqueuse parentérale injectable multidose selon l'une quelconque des revendications 1 à 7, pour utilisation dans le traitement du diabète.

9. Composition pharmaceutique aqueuse parentérale injectable multidose selon l'une quelconque des revendications 1 à 7, pour utilisation dans le traitement de l'adipose.
